(19) **Europäisches Patentamt / European Patent Office / Office européen des brevets**

(11) **EP 2 570 193 B1**

(12) **EUROPEAN PATENT SPECIFICATION**

(45) Date of publication and mention
of the grant of the patent:
**27.08.2014 Bulletin 2014/35**

(51) Int Cl.:
***B05B 1/34*** *(2006.01)*    ***B05B 7/24*** *(2006.01)*
***B65D 83/38*** *(2006.01)*

(21) Application number: **12184248.8**

(22) Date of filing: **13.09.2012**

(54) **Spray nozzle for dispensing a fluid and sprayer comprising such a spray nozzle**

Sprühdüse zum Abgeben einer Flüssigkeit und Sprüheinrichtung, die eine solche Sprühdüse umfasst

Buse de pulvérisation pour distribuer un fluide et pulvérisateur comportant une telle buse de pulvérisation

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.09.2011 EP 11007522**

(43) Date of publication of application:
**20.03.2013 Bulletin 2013/12**

(73) Proprietor: **The Procter & Gamble Company
Cincinnati, OH 45202 (US)**

(72) Inventor: **Dal Bò, Paolo
60322 Frankfurt am Main (DE)**

(74) Representative: **Töpert, Verena Clarita
Procter & Gamble Service GmbH
Patent Department
Berliner Allee 65
64274 Darmstadt (DE)**

(56) References cited:
**WO-A1-03/061839        GB-A- 2 293 336
US-A- 5 335 858          US-A1- 2007 018 017
US-A1- 2009 084 870**

## Description

FIELD OF THE INVENTION

The present invention relates to a spray nozzle for dispensing a fluid comprising a first element, preferably a nozzle cup, and a second element, preferably a pin, said first and second element forming an assembly comprising a fluid chamber, preferably a ring chamber, for receiving the fluid, at least one feeding channel for feeding the fluid from the fluid chamber radially inward into a swirl chamber and an outlet channel with an entrance end facing the swirl chamber and an exit end for discharging the fluid to the environment of the spray nozzle. The present invention further relates to sprayers comprising such a spray nozzle.

BACKGROUND OF THE INVENTION

Spray nozzles for dispensing fluids are well known in the state of the art. Usually, the known spray nozzles comprise a fluid chamber, at least one feeding channel for feeding the fluid from the fluid chamber radially inward into a swirl chamber and an outlet channel with an entrance end facing the swirl chamber and an exit end for discharging the fluid to the environment of the spray nozzle. The cross-section of the outlet channel has a constant form and a constant cross-sectional area in the flow direction of the fluid.

The known spray nozzles for dispensing fluids have proved themselves. However, a relatively high pump pressure is necessary, especially if highly viscous or tough-flowing fluids have to be dispensed. Above this, it is desirable to reduce energy dissipation within the known spray nozzles in order to maximize discharge velocity of the fluid. Further, the known spray nozzles do not allow a flexible adjustment of the spray pattern, the volume flow rate and so forth. Furthermore, the production and prototyping of the known spray nozzles may be complicated.

It is therefore an object of the present invention to provide a spray nozzle for dispensing a fluid, the spray nozzle reducing the necessary pump pressure and provides a decrease of energy dissipation. In other words, it is an object of the present invention to provide a spray nozzle generating the same particle size as conventional nozzles at a lower pressure or generating a spray with a smaller particle size at the same pressure. It is a further object of the present invention to provide a sprayer with a spray nozzle according to the invention with reduced pump pressure and reduced energy dissipation within the spray nozzle. In other words, it is an object of the present invention to provide a spray with the same particle size at a lower pressure, or to generate a spray with smaller particle size at the same pressure. In other words, it is an object of the present invention to increase the efficiency of the spray nozzle.

In particular, in a pressure-swirl atomizer, a swirling motion is imparted on the fluid, leading under centrifugal acceleration $\left(a_c = \frac{v_y^2}{r}\right)$ to a spread of the fluid in the form of a hollow cone when the fluid is discharged to the environment. The centrifugal acceleration ($a_c$) - being dependent on the square of the rotational velocity ($v_y$) - is responsible for the atomization of the fluid, i.e. for generating fluid particles with a certain size. With decreasing nozzle dimensions (e.g. decreasing diameter of the exit end of the outlet channel or of the feeding channels) the energy dissipation through the nozzle increases, thus, the rotational velocity ($v_y$) decreases. Thus, in case the diameter of the exit end of the outlet channel is relatively small, centrifugal acceleration decreases and the provision of small particle sizes at low pressure is not sufficient. Thus, higher nozzle efficiency is required for spray nozzles, in particular for nezzles having a relatively small diameter of the exit end of the outlet channel.

SUMMARY OF THE INVENTION

The above-mentioned problem is solved by a spray nozzle and a sprayer as described in claims 1 and 15, respectively. Preferred and advantageous embodiments of the invention are described in the subclaims.

The present invention is directed to a spray nozzle for dispensing a fluid. The spray nozzle comprises a first and a second element which form an assembly. Preferably, the first element is a nozzle cup and the second element is a pin. The assembly comprises a fluid chamber for receiving the fluid. It is preferred, if the fluid chamber is configured as a ring chamber, it is in fluid connection with a fluid storage chamber for storing the fluid. Said ring chamber is connecting with the swirl chamber, which will be described later. The spray nozzle further comprises at least one feeding channel for feeding the fluid from the fluid chamber radially inward into a swirl chamber. Usually, one feeding channel may be sufficient, however, it has been found out that two feeding channels in a symmetrical arrangement effect a better production of swirl within the swirl chamber and consequently a more symmetrical spray pattern. It is further preferred, if the feeding channel tangentially leads into the swirl chamber. According to the invention, there is further provided an outlet channel. The outlet channel has an entrance end facing the swirl chamber and an exit end for discharging the fluid to the environment of the spray nozzle. In order to provide a spray nozzle needing a relatively low pump pressure and which is applicable even if highly viscous or tough-flowing fluids have to be dispensed, the outlet channel tapers in the flow direction of the fluid. It has been found out that energy dissipation within the spray nozzle could be reduced by using the tapered outlet channel wherein the degree of tapering is either constant or the degree of tapering decreases in the flow direction. Further, the outlet channel tapering in the flow direction has positive effects on the spray pattern. The ratio of the sum of the cross-sectional areas of the at least one feeding channel at their exit end to the cross-sectional area of the exit end of the outlet channel is between about 1.5 and 2.7.

In an embodiment of the spray nozzle the degree of tapering is constant in the flow direction and at least a tapering portion of the outlet channel or the whole outlet channel has the form of a truncated cone or a truncated pyramid.

In a further embodiment of the spray nozzle the degree of tapering decreases in the flow direction. It has been found out that the spray nozzle could further be improved with regard to the advantageous effects mentioned above by decreasing the degree of tapering in the flow direction. It especially has a positive effect on the spray pattern and the spray angle without having a negative influence on the pressure drop, i.e. energy dissipation within the nozzle and the pump pressure necessary to dispense the fluid. Very good results have been achieved by providing an inner face of the outlet channel which is curved in the flow direction, this modification being further preferred in this embodiment. In this connection it is further preferred if at least a tapering portion of the outlet channel or the whole outlet channel more has the form of a truncated hyperboloid of revolution.

Basically, the outlet channel may taper stepwise in the flow direction. However, in a preferred embodiment of the spray nozzle the outlet channel tapers steadily, thereby achieving a further reduction of the necessary pump pressure and a further lowering of energy dissipation within the nozzle.

In principle, a tapering portion of the outlet channel may be provided anywhere along the outlet channel to at least partially achieve the advantages mentioned above. However, in a further preferred embodiment of the spray nozzle according to the invention the outlet channel comprises a tapering portion within which the outlet channel tapers in the flow direction, said tapering portion abutting the exit end of the outlet channel. In other words, the end of the tapering portion forms the exit end of the outlet channel, so that the outlet channel tapers in the flow direction until it reaches the exit end of it. A further reduction of the necessary pump pressure and a further lowering of energy dissipation within the nozzle could be achieved hereby. Above this, the described modification improves the spray pattern.

In a further preferred embodiment of the spray nozzle the tapering portion does not only abut the exit end of the outlet channel, the tapering portion rather abuts the entrance end of the outlet channel, as well. In other words, the one end of the tapering portion forms the exit end of the outlet channel while the other end of the tapering portion forms the entrance end of the outlet channel, so that the tapering portion runs from the one end of the outlet channel to the other end of the outlet channel. Thus, the whole outlet channel tapers in the flow direction. It has been found out that the spray nozzle could thereby further be improved with regard to the advantageous effects mentioned above.

Due to the production method of the spray nozzle, the edge surrounding the exit end of the outlet channel is rounded, so that it has a radius. In an advantageous embodiment of the spray nozzle the edge surrounding the exit end has a radius being smaller than 0.03 mm, preferably smaller than 0.02 mm. It has been found out that the spray nozzle could further be improved with regard to the advantageous effects by limiting the radius of the edge accordingly.

In an embodiment of the spray nozzle the exit end of the outlet channel has a maximum diameter between about 0.1 mm and about 0.8 mm, preferably between about 0.1 mm and about 0.25 mm, more preferably between about 0.1 mm and about 0.2 mm and still more preferably between about 0.12 mm and about 0.15 mm. For a flow rate (for the fluid water is considered) between 0.10 and 0.15 ml/s at 1 bar or approximately 0.20 and 0.35 ml/s at 9 bar, it results to be advantageous an exit diameter between 0.1 mm and 0.15 mm to achieve an average particle size by volume ($D_{50}$), which is equal or lower than 100 micrometer at 1 bar and equal or lower than 60 micrometer at 9 bar, or preferably equal or lower than 95 micrometer at 1 bar and equal or lower than 55 micrometer at 9 bar. For spraying higher particle size at the same pressure, the diameter has to be bigger and proportionally with it, the channel geometry. A bigger geometry of the nozzle, which sprays a higher flow rate, would be even more advantageous in order to achieve smaller particle size at the same pressure and fluid characteristics and would allow the production of even smaller particle size that the ones mentioned above.

In a further embodiment of the spray nozzle the inner face of the outlet channel includes an angle, said angle varying between 70° and 130°, preferably between 80° and 120°, more preferably between 80° and 110°, in order to achieve a positive effect on the spray pattern and the minimum pump pressure necessary to dispense the fluid.

In an embodiment of the spray nozzle the feeding channel comprises a first section and a second section following the first section in the flow direction and abutting the swirl chamber. In order to keep up the pressure within the nozzle and to avoid a pressure drop, i.e. energy dissipation, respectively, the width of the first section decreases in the flow direction. In this embodiment, the width of the second section is constant or decreases to a lesser extent than the width of the first section in the flow direction. In this embodiment, it is further preferred if the side walls of the first section includes an angle, said angle being subdivided into a first angle and a second angle by a centerline of the second section, the maximum difference between the first angle and the second angle being 10°, more preferably 5° or 1°. In the ideal case the first angle corresponds to the second angle. It has been found out that the positive effects mentioned before, especially decreasing the energy dissipation within the nozzle and creating of the swirl within the swirl chamber, could further be enhanced.

In order to further enhance the positive effects of the invention as mentioned before, in an embodiment of the spray nozzle the length of the second section in the flow direction is equal to or smaller than the width of the second section.

In order to avoid the pressure drop, i.e. energy dissipation within the nozzle, thereby reducing the minimum pump pressure of the spray nozzle, in an embodiment of the spray nozzle the height of the first or/and second section is decreasing in the flow direction. In this case it is also preferred if the degree of height reduction is constant, decreasing or increasing in the flow direction. If the degree of height reduction is decreasing or increasing it is further preferred if the bottom of the second section and the second element are curved and that the bottom of the second section has a flat outer surface.

In an embodiment of the spray nozzle the width of the second section is substantially equal to the height of the second section. This further enhances the positive effects of the invention as mentioned above.

In an embodiment of the spray nozzle the ratio of the diameter of the swirl chamber to the diameter of the exit end is about 2.5 to about 3.5. Using water as a fluid, this ratio provides a particle size ($D_{32}$) of about 23 $\mu$m.

In order to further enhance the positive effects of the invention as mentioned before, in an embodiment of the spray nozzle the ratio of the sum of the cross-sectional areas of the at least one feeding channel at their exit end, i.e. where the feeding channels abut the swirl chamber, to the cross-sectional area of the exit end of the outlet channel is preferably between about 1.7 and about 2.6.

In an embodiment of the spray nozzle the bottom of the first element exerts a pretension against the flow direction of the fluid of about 0.5 N to about 1.5 N, preferably of about 1 N. In other words, during the assembly of the spray nozzle, i.e. when the second element is inserted into the first element a bending of the bottom of the first element to a flat position occurs, thereby generating a pretension of about 0.5 N to about 1.5 N, preferably of about 1 N against the second element. This pretension assures adhesion of the first element to the second element when fluid is dispensed at high pressure, e.g. of about 20 bar which corresponds to 0.5 N.

Preferably, the bottom of the first element is conical in longitudinal direction forming with the second element a contact area which is defined by the penetration of the second element during the assembly, which generates the pretension between the first element and the second element due slightly bending the bottom of the first element in longitudinal direction.

In order to facilitate the assembly of the spray nozzle, in an embodiment the spray nozzle is assembled from the first element comprising protrusions for forming the side walls of the feeding channels and grooves between the protrusions and the second element being supported on the protrusions and covering the grooves in order to form the feeding channels. In order to support the second element, the protrusions comprise a support surface.

Usually the second element, preferably a pin, being supported on the protrusions and covering the grooves in order to form the feeding channels is part of the sprayer for which the nozzle is intended to be used.

In order to further facilitate assembly the second element is a separated part which can be jammed or welded into the first element. In this embodiment, to facilitate assembly, the second element can be jammed or welded into the first element and is molded together with the first element in one single part and connected by a flexible connecting piece.

Due to the production method of the spray nozzle, the transition region between the support surface of the protrusions and the surface of the protrusions facing the feeding channel may be rounded, so that it has a radius. In an embodiment of the spray nozzle the ratio of the radius between the support surface of the protrusion and the surface of the protrusion facing the feeding channel to the width of the feeding channel is equal to or less than 1/3, more preferably equal to or less than 1/4, most preferably equal to or less than 1/5, in order to achieve a compact cross-sectional form and to reduce the pressure drop, i.e. energy dissipation within the nozzle.

In order to facilitate the assembly of the spray nozzle and to ensure a tight support of the second element on the first element, in an embodiment of the spray nozzle one of the first and second element comprises an elastic portion, said elastic portion being elastically deformed by the other element when the elements are assembled. In other words, one of the first and second elements is elastically pressed against the other of the first and second elements when the elements are assembled. This pretension further avoids separation of the first and second element if high pressures occur within the spray nozzle.

In an embodiment of the spray nozzle the protrusions or/and the section of the first element carrying the protrusions form the elastic portion. The section of the first element carrying the protrusions is preferably a bottom of a cup-like first element being fixed to a surrounding wall of the cup-like first element. In this case, it is further preferred if the bottom is curved or convex towards the second element, before the first and second element are assembled. Said bottom may for example be more elastic than the surrounding wall.

In order to achieve an easily adjustable and flexible spray nozzle with regard to the pump pressure, the volumetric flow, the spray pattern, the spray angle or the like, in an embodiment of the spray nozzle the assembled first and second element are movable relative to each other into different relative positions thereby elastically changing the form, dimensions or/and justification of the feeding channels or/and the swirl chamber. It is further preferred, if the elements are lockable in their different relative positions, so that the change in pump pressure, volumetric flow, spray pattern, spray angle or the like could be kept up without the need to hold both elements in their relative position manually.

In order to further facilitate the production of the spray nozzle and the handling of the same during the assembling, in an embodiment of the spray nozzle the first element and the second element are connected via a flexible connecting piece. Thus the two elements may be moved relative to each other during the assembling without the risk that one or the other element gets lost. The flexible connecting piece it preferably formed by a strip. It is further preferred, if the connecting piece is integrally formed or molded with the first and second element or at least a part of the first and second element in order to facilitate the production of the spray nozzle.

In an embodiment of the spray nozzle an outlet layer with a first hole, a channel layer with a second hole and slots and an inlet layer with holes are provided, said layers being sandwiched such that the first hole forms the outlet channel, the second hole forms the swirl chamber, the slots form the feeding channels and the holes in the inlet layer form inlet holes for feeding the fluid from the fluid chamber into the feeding channels. The layers could for example be integrally formed, e. g. by galvanization.

In order to allow a quick cleaning and prototyping of the spray nozzle, in an embodiment of the spray nozzle the layers are separable from each other or/and each of the layer is replaceable. Above this, this modification allows to produce a plurality of combinations of layers out of a few prefabricated layers without the need to provide the same number of single layers. The separable or/and replaceable layers could for example be provided in the form of thin discs.

In order to provide a spray nozzle being flexible with regard to the pump pressure, the volumetric flow, the spray pattern, the spray angle or the like, in an embodiment of the spray nozzle there is provided an overlapping area between the inlet holes and the feeding channels, in order to feed the fluid through the inlet holes into the feeding channels. The size of the overlapping area or/and the distance between the overlapping area and the swirl chamber is preferably adjustable. By changing the size of the overlapping area or/and the distance between the overlapping area and the swirl chamber, the spray pattern or the like could be easily changed. In order to facilitate the handling of the spray nozzle, the inlet layer and the channel layer are more preferably moveable, most preferably rotatable, relative to each other in order to adjust the size of the overlapping area or/and the distance between the overlapping area and the swirl chamber. As alternative in order to further facilitate the handling of the spray nozzle, the inlet layer and the channel layer are more preferably moveable, by snapping them, rotating them on their axis to adjust the size of the overlapping area or shifting them by lateral movement to exchange a layer.

In an embodiment, the spray nozzle is made of a plastic material selected from the following list: polyoxymethylene, polypropylene, polyethylene, polystyrene, acrylonitrile butadiene styrene, silicone, polyamide, polyethylene terephthalate or mixtures thereof. Further, the spray nozzle can additionally comprise an elastomer.

In an embodiment, the spray nozzle is made by an injection molding process. This provides a very precise molding process and enables to manufacture an exit end of the outlet channel with a diameter smaller that 0.25 mm and, in addition, a relatively sharp angle at the exit end with a radius of less than about 0.03 mm or less than about 0.02 mm.

The sprayer according to the invention, e.g. an easy sprayer, a continuous sprayer, a squeeze sprayer, a sprayer with a pressurized fluid storage container or a low energy electrical sprayer, comprises an embodiment of the spray nozzle according to the invention. The advantages mentioned in connection with the spray nozzle apply mutatis mutandis.

In an embodiment of the sprayer the sprayer is a hand operated sprayer, e.g. a trigger sprayer, the sprayer preferably comprising a fluid storage container being manually squeezable or another manually actuable pumping device. Alternatively, the sprayer is an electrically driven sprayer. In both cases, due to the advantages of the spray nozzle there is no need to apply a high pump force, so that actuation is easier and less energy consuming.

The spray nozzle may be used with different kinds of fluids in industry and/or the marketplace, including "consumer care products." Such consumer care products offered by the consumer care industry include, for example and without limitation, soft surface cleaners, hard surface cleaners, glass cleaners, ceramic tile cleaners, toilet bowl cleaners, wood cleaners, multi-surface cleaners, surface disinfectants, dishwashing compositions, laundry detergents and stain treatments, fabric conditioners, fabric dyes, surface protectants, surface disinfectants, motor vehicle surface treatments, and other like consumer products. Consumer care products may also be for household or home care use as well as for professional, commercial and/or industrial use. The consumer product industry may also produce "personal care products" comprising fluids including, for example and without limitation, hair treatment products including mousse, hair spray, styling gels, shampoo, hair conditioner (leave-in or rinse-out), cream rinse, hair dye, hair coloring product, hair shine product, hair serum, hair anti-frizz product, hair split-end repair products, permanent waving solution, antidandruff formulation; bath gels, shower gels, body washes, facial cleaners, skin care products including sunscreen and sun block lotions, skin conditioner, moisturizers, perfumed and non-perfumed underarm and body antiperspirant compositions and deodorants, soaps, body scrubs, exfoliants, astringent, scrubbing lotions, depilatories, shaving products, preshaving products, after shaving products, toothpaste or dentifrice. Other applications of the spray nozzle can apply to other fluids in other categories of products such as: indoor and/or outdoor insecticide and herbicide products applied indoors or outdoors; the medicinal industry having product forms including, for example and without limitation, medications, medicaments and treatments which include, ointments, creams, lotions, and the like; and, the food and beverage industry.

BRIEF DESCRIPTION OF THE DRAWINGS

Preferred embodiments of the present invention will now be described, by way of example only, with reference to the drawings in which

Fig. 1 shows a cross-sectional side-view of a first embodiment of the spray nozzle;

Fig. 2 shows a cross-sectional view along line A-A in Fig. 1;

Fig. 3 shows a cross-sectional view along line B-B in Fig. 2;

Fig. 4 shows the enlarged section A of Fig. 1;

Fig. 5 shows the enlarged section A of Fig. 1 with a first modification;

Fig. 6 shows a schematic view of a second embodiment of the spray nozzle;

Fig. 7 shows a schematic view of a third embodiment of the spray nozzle;

Fig. 8 shows a correlation curve of rotational velocity vs. particle size;

Fig. 9 shows a correlation curve of nozzle efficiency vs. particle size;

Fig. 10 shows a particle size distribution of an embodiment of the invention;

Fig. 11 shows a particle size distribution of a spray nozzle according to the state of the art;

Fig. 12 shows a cross-sectional side-view of a nozzle according to the state of the art; and

Fig. 13 shows a cross-sectional view of the spray nozzle according to Fig. 12.

DETAILED DESCRIPTION OF THE INVENTION

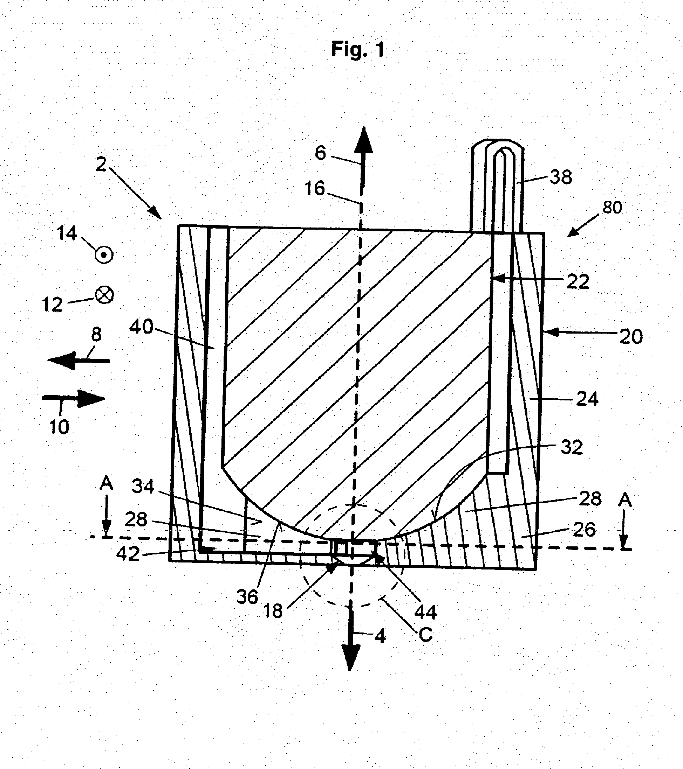

Fig. 1 to 4 show views of a first embodiment of the spray nozzle 2 for dispensing a fluid. In the figures, the opposing longitudinal directions 4, 6, the opposing radial directions 8, 10 and the opposing circumferential directions 12, 14 of the spray nozzle are indicated by corresponding arrows. The longitudinal axis 16 of the spray nozzle 2 extends in the longitudinal directions 4, 6, said longitudinal axis 16 further forming the centre axis of the outlet channel 18.

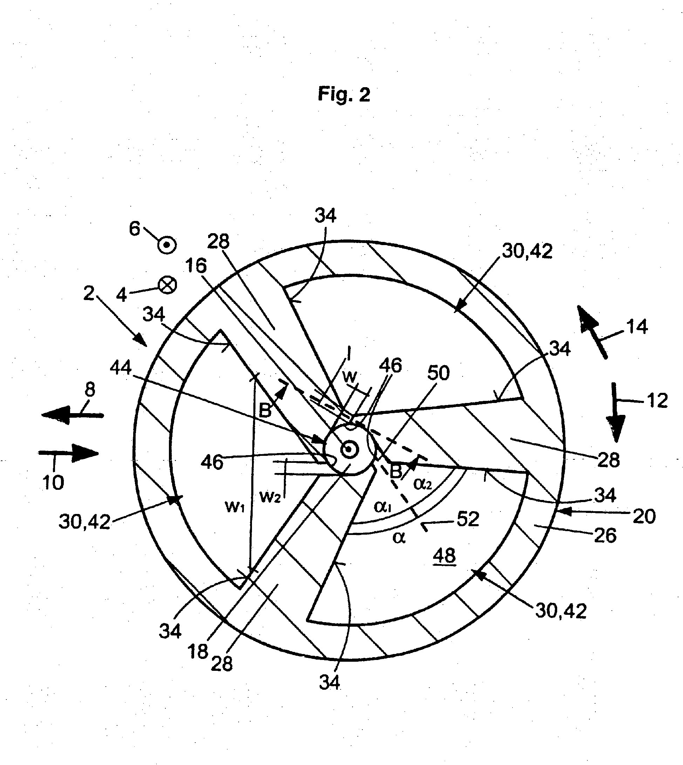

The spray nozzle 2 is assembled from a first element 20 and a second element 22 thereby forming an assembly 80. The first element 20 is a nozzle cup 20, i.e. having a cup-like structure with a first section 24 extending in the circumferential directions 12, 14 and forming a surrounding wall and a second section 26 forming the bottom 26. The second section 26 further comprises protrusions 28, said rib-like protrusions 28 extending in the longitudinal direction 6 and in the radial directions 8, 10. As can be best seen in Fig. 2, there are provided grooves 30 in the circumferential directions 12, 14 between the protrusions 28, said grooves being provided to form the feeding channels 42 as will be described later. The protrusions 28 comprise an upper surface serving as a support surface 32 for supporting the second element 22, said support surface 32 facing the second element 22. Further, the protrusions 28 comprise side surfaces 34 facing the grooves 30 and feeding channels 42, respectively.

The second element 22 may be a pin 22 basically having a cylindrical form with a front face 36, said front face 36 bulging out in the longitudinal direction 4. In this embodiment, the front face 36 has a form of a spherical cap. The second element 22 is inserted into the first element 20, so that the front face 36 is supported on the support surfaces 32 of the protrusions 28. In this connection it should be mentioned, that the second element 22 may also be formed by a ball, which is pressed or clipped into the first element 20. Independent of the chosen form of the second element 22, it is preferred if the second element 22 could be snapped or clicked into its place within the first element 20, even if corresponding notches, snaps or the like for providing a form-fit or/and a force-fit are not shown in the figures.

The first element 20 and the second element 22 may be connected via a flexible connecting piece 38, which - in this case - is formed by a strip. The connecting piece 38 is integrally formed or molded with the second element 22 and at least the first section 24 of the first element 20. Even the second section 26 of the first element 20 may be integrally formed or molded with the first section 24 of the first element 20 and consist of the same material. However, in this case the second section 26 has been subsequently fastened to the first section 24 since the second section 26 is made of a different material, as will be described hereinafter. Irrespective of the second section 26 being integrally formed with the first section 24 or not, the first element 20 comprises an elastic portion.

As already indicated above, the first element 20 is at least partially made of an elastic material being more elastic than the material of the second element 22. In this case, the second section 26 of the first element 20 with its protrusions 28 and its bottom section 26 carrying said protrusions 28 is made of the elastic material, said elastic material being more elastic than the material of the second element 22 and more elastic than the material of the first section 24 of the first element 20. Thus, the aforementioned elastic portion of the first element 20 is essentially formed of the protrusions 28 and its bottom section carrying said protrusions 28. The elastic portion of the first element 20 is elastically deformed by the second element 22 when the elements 20, 22 are assembled.

Further, the bottom 26, i.e. second section 26, of the first element 20 exerts a pretension against the flow direction of the fluid of about 0.5 N to about 1.5 N, preferably of about 1 N. In other words, during the assembly of the spray nozzle 2, i.e. when the second element 22 is inserted into the first element 20 a bending of the bottom 26 of the first element 20 to a flat position occurs, thereby generating that pretension against the second element 22. This pretension assures adhesion of the first element 20 to the second element 22 when fluid is dispensed at high pressure.

Even if the pre-assembled state is not shown, it is preferred if the bottom section carrying said protrusions 28 is curved or convex towards the second element 22 and in the longitudinal direction 6 before the first and second element 20, 22 are assembled.

In one example, the spray nozzle 2 is assembled by inserting the pin 22 into the nozzle cup 20 in the longitudinal direction 4 as shown in Fig. 1, thereby creating a fluid chamber 40, feeding channels 42 and a swirl chamber 44, while the outlet channel 18 is already provided in the second section 26 of the nozzle cup 20. The fluid chamber 40 is positioned in the radial directions 8, 10 between the first section 24 of the nozzle cup 20 and the pin 22, so that the fluid chamber 40 is formed as a ring chamber. The fluid chamber 40 receives the fluid to be dispensed from a fluid storage chamber or container, which is not shown in the drawings. In the longitudinal direction 4 the fluid chamber 40 abuts the radial outer ends of the feeding channels 42, so that there is a fluid connection between the fluid chamber 40 and the feeding channels 42.

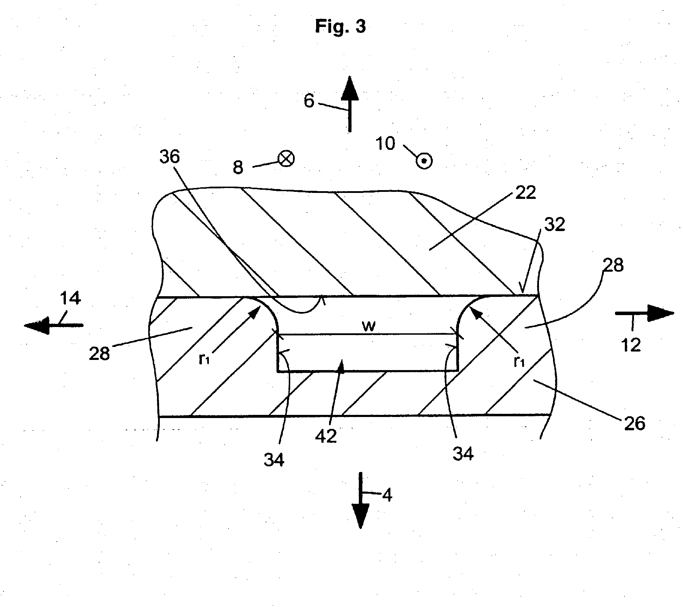

As can especially be seen in Fig. 2, the feeding channels 42 are extending radially inward to an exit end 46 of the feeding channels 42, where the feeding channels 42 abut the swirl chamber 44, so that the fluid may be fed from the fluid chamber 40 via the feeding channels 42 into the swirl chamber 44. As shown in Fig. 3, the feeding channels 42 are limited in the circumferential directions 12, 14 by the side surfaces 34 of the protrusions 28, in the longitudinal direction 6 by the front face 36 of the second element 22, said second element 22 covering the grooves 30 to form the feeding channels 42, and in the longitudinal direction 4 by the bottom of the second section 26 carrying the protrusions 28.

In Fig 2, the feeding channels 42 comprise a first section 48 abutting the fluid chamber 40 and a second section 50 following the first section 48 in the flow direction and radial direction 10, respectively. The second section 50 abuts the swirl chamber 44 with the exit end 46. As shown in Fig. 2, the width w1 of the first section 48 decreases in the flow direction and the radial direction 10. In contrast to this, the width w2 of the second section 50 is constant or decreases to a lesser extent than the first section 48 in the flow direction and radial direction 10.

The protrusions 28, which form the side walls of the first sections 48, include an angle $\alpha$, between the protrusions' side walls as shown. In Fig. 2, there is further indicated a centerline 52 of the second section 50 extending in the radial directions 8, 10. Said centerline 52 subdivides the angle $\alpha$ into a first angle $\alpha1$ and a second angle $\alpha2$. The maximum difference between the first angle $\alpha1$ and the second angle $\alpha2$ is 10°, more preferably 5° or 1°, most preferably 0°. Due to the bulged out front face 36 of the second element 22, the height h of the first section 48 or/and second section 50 of the feeding channels 42 decreases in the flow direction and the radial direction 10. Further, the length 1 of the second section 50 in the flow direction and the radial direction 10 is equal to or smaller than the width w2 of the second section 50. In addition, the width w2 of the second section 50 is equal to the height h of the second section 50.

As shown in Fig. 3, in the transition region between the support surfaces 32 and the side surfaces 34 the protrusions 28 comprise a radius r1. In order to have a compact cross-sectional form, the ratio of the radius r1 to the width w, e. g. w1 or w2, of the feeding channel 42 is equal to or less than 1/3, more preferably equal to or less than 1/4, most preferably equal to or less than 1/5.

Even if the first element 20 and the second element 22 are assembled, they are still movable relative to each other into different relative positions. In the shown embodiment, the elements 20 and 22 may be moved in the longitudinal direction 4, 6 relative to each other. By this relative movement the form, dimensions or/and justification of the feeding channels 42 or/and the swirl chamber 44 is changed by elastically deforming the protrusions 28 or/and the bottom of second section 26 of the first element 20, i.e. by elastically deforming the elastic portion of the first element 20. In other words, it is easy to change the behavior of the spray nozzle 2. Further, there are provided means (not shown) for locking the elements 20, 22 in their different relative positions.

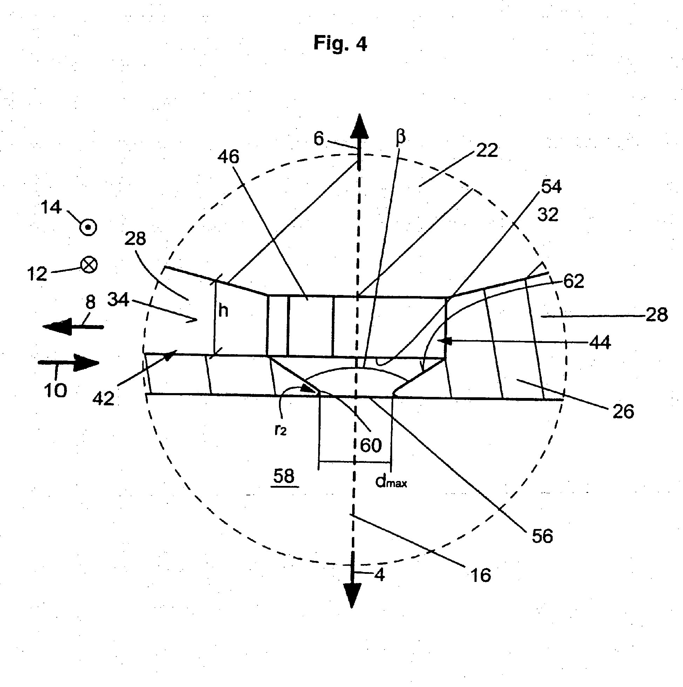

With reference to Fig. 4, the afore-mentioned outlet channel 18 in the second section 26 of the first element 20 comprises an entrance end 54 facing the swirl chamber 44 in the longitudinal direction 6 and an exit end 56 for discharging the fluid to the environment 58 of the spray nozzle 2 and the sprayer, respectively, in the longitudinal direction 4. The outlet channel 18 tapers steadily in the flow direction and the longitudinal direction 4. Thus, the outlet channel 18 comprises at least one tapering portion, i.e. the outlet channel 18 is tapered in at least part along the length of the outlet channel 18 toward the exit. The tapering may be continuous or in steps, and may be angled or curved. In the shown embodiment, the tapering portion abuts the exit end 56 as well as the entrance end 54 of the outlet channel 18, so that the whole outlet channel tapers in the flow direction. The edge 60 surrounding the exit end 56 has a radius $r_2$. The radius $r_2$ is smaller than 0.03 mm, preferably smaller than 0.02 mm.

Further, the exit end 56 has a maximum diameter between 0.12 mm and 0.15 mm and more preferably a diameter about 0.14 mm with a corresponding maximum cross-sectional area to achieve an average particle size by volume ($D_{50}$) with a flow rate higher than 0.24 g/s at 9 bar (for the fluid water is considered), which is equal or lower than 60 $\mu$m, or preferably equal or lower than 50 $\mu$m, or more preferably equal or lower than 45 $\mu$m. This diameter further achieves an average particle size by volume ($D_{32}$) which is equal or lower than 50 $\mu$m, or preferably equal or lower than 45 $\mu$m, or more preferably equal or lower than 40 $\mu$m. The average percentage of particles having a diameter smaller than 10 $\mu$m (%< 10 $\mu$m) is less than 2%, preferably less than 1.5%, more preferably less than 1%.

A bigger geometry of the nozzle having a diameter ($d_{max}$) about 0.8 mm and, thus, providing a higher flow rate, e.g. higher than 3.2 g/s at 2 bar, achieves an average particle size by volume ($D_{50}$) (for the fluid water is considered) which is equal or lower than 120 $\mu$m, or preferably equal or lower than 115 $\mu$m. This diameter further achieves an average particle size by volume ($D_{32}$) which is equal or lower than 100 $\mu$m, or preferably equal or lower than 96 $\mu$m. The average percentage of particles having a diameter smaller than 10 $\mu$m (%< 10 $\mu$m) is less than 1.5%, preferably less than 1%, more preferably less than 0.5%.

Above this, the outlet channel 18 has an inner face 62 surrounding the outlet channel 18 and limiting the same in the radial direction 8. The inner face 62 of the outlet channel 18 includes an angle $\beta$, said angle $\beta$ preferably varying between 70° and 130°, preferably between 80° and 120°, more preferably between 80° and 110°.

As shown in Fig. 4, the degree of tapering of the outlet channel 18 is constant in the flow direction and the longitudinal direction 4. In the shown embodiment this is achieved by at least a tapering portion of the outlet channel 18 or the whole outlet channel 18 having the form of a truncated cone or a truncated pyramid. It has further been found out, that the pressure drop, i.e. energy dissipation in the spray nozzle 2 could be reduced and a further reduction of the minimum pump pressure for dispensing the fluid could be achieved by adjusting the ratio of the sum of the cross-sectional areas of the feeding channels 42 at their exit end 46 to the cross-sectional area of the exit end 56 of the outlet channel 18. This ratio is between about 1.5 and about 2.7, preferably between about 1.7 and about 2.6. Further, the ratio of the diameter $d_s$ of the swirl chamber 44 to the diameter $d_{max}$ of the

exit end 56 of the outlet channel 18 is about 2.5 to about 3.5.

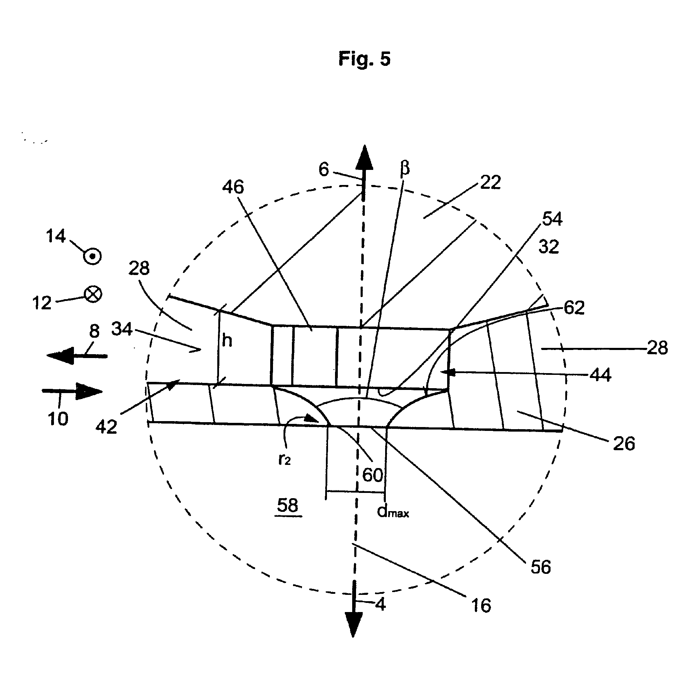

Fig. 5 shows the enlarged section A of Fig. 1 with a first modification. In the following only the differences will be described, the same reference signs will be used for similar or the same components and the above description of the first embodiment applies accordingly in this regard.

In contrast to the outlet channel 18 described with reference to Fig. 1 to 4, the degree of tapering of outlet channel 18 according to Fig. 5 decreases in the flow direction and the longitudinal direction 4. This is achieved by providing an inner face 62 of the outlet channel 18 being curved in the flow direction and the longitudinal direction 4. In the embodiment according to Fig. 5, at least the tapering portion of the outlet channel 18 or the whole outlet channel 18 has the form of a truncated hyperboloid of revolution.

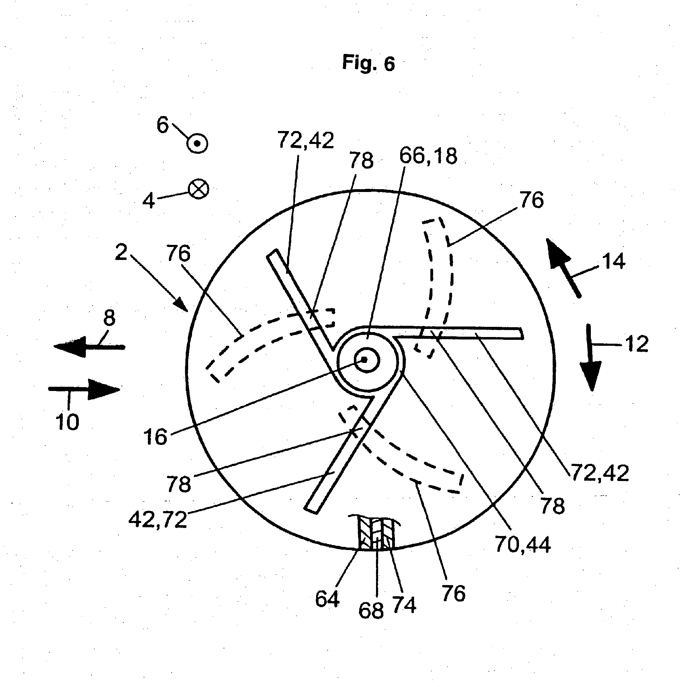

Fig. 6 shows a second embodiment of the spray nozzle according to the invention. Since the second embodiment at least partially corresponds to the first embodiment according to Fig. 1 to 5, in the following only the differences will be described, the same reference signs will be used for similar or the same components and the above description of the first embodiment applies accordingly in this regard.

The spray nozzle 2 according to Fig. 6 comprises at least three layers, i.e. an outlet layer 64 with a first hole 66, a channel layer 68 with a second hole 70 and slots 72 and an inlet layer 74 with slot-like holes 76, said layers 64, 68 and 74 being sandwiched, while the inlet layer 74 is shown in a transparent manner in Fig. 6 to increase the intelligibility of the drawing. Being sandwiched this way, the first hole 66 forms the outlet channel 18, the second hole 70 forms the swirl chamber 44, the slots 72 form the feeding channels 42 and the holes 76 in the inlet layer form inlet holes for feeding the fluid from the fluid chamber 40 into the feeding channels 42. In the shown embodiment, the layers 64, 68 and 74 are separable from each other and each of the layers 64, 68 and 74 could be replaced, so that the layers 64, 68 and 74 could also be regarded as separate discs with corresponding slots and holes.

As shown in Fig. 6, there is provided an overlapping area 78 between the inlet holes 76 and the feeding channels 42 when viewed in the longitudinal direction 4. The inlet layer 74 and the channel layer 68 are moveable - in this case rotatable around the longitudinal axis 16 - relative to each other, while the inlet holes 76 and the feeding channels 42 are formed such that, the distance between the overlapping area 78 and the swirl chamber 44 could be reduced by rotating the inlet layer 74 relative to the channel layer 68 in the circumferential direction 14 and could be enlarged by rotating the inlet layer 74 relative to the channel layer 68 in the circumferential direction 12. Thus, the distance between the overlapping area 78 and the swirl chamber 44 is adjustable.

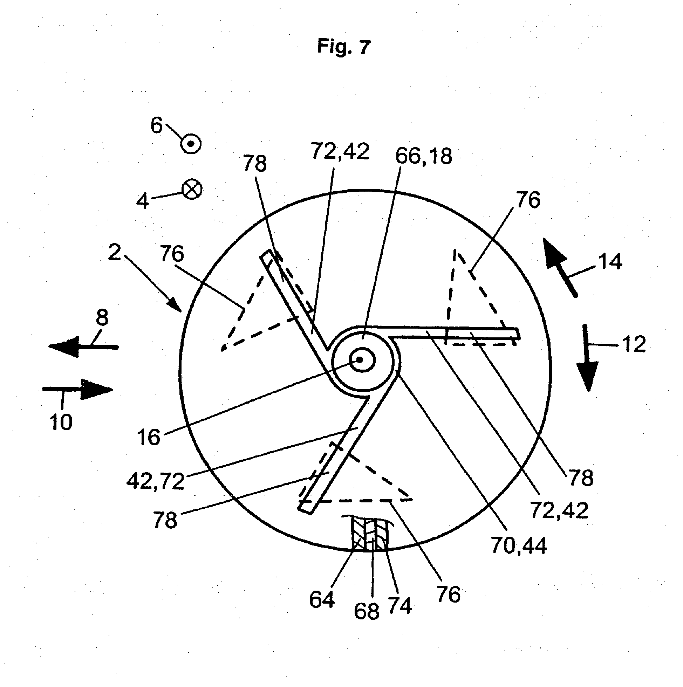

Fig. 7 shows a third embodiment of the spray nozzle 2 according to the invention. Since the third embodiment at least partially corresponds to the second embodiment according to Fig. 6, in the following only the differences will be described, the same reference signs will be used for similar or the same components and the above description of the first and second embodiment applies accordingly in this regard.

In contrast to the second embodiment, the inlet holes 76 and the feeding channels 42 of the third embodiment are formed such that, the size of the overlapping area 78 could be reduced by rotating the inlet layer 74 relative to the channel layer 68 in the circumferential direction 12 and could be enlarged by rotating the inlet layer 74 relative to the channel layer 68 in the circumferential direction 14. Thus, the size of the overlapping area 78 is adjustable.

It should be mentioned that the principles of the second and third embodiment could also be advantageously combined in a single spray nozzle 2, so that the size of the overlapping area 78 as well as the distance between the overlapping area 78 and the swirl chamber 44 could be adjusted by a relative movement between the inlet layer 74 and the channel layer 68.

The spray nozzle 2 is made of a plastic material, e.g. polyoxymethylene, polypropylene, polyethylene, polystyrene, acrylonitrile butadiene styrene, silicone, polyamide, polyethylene terephthalate or mixtures thereof. Further, the spray nozzle can additionally comprise an elastomer.

According to the invention, the spray nozzle 2 should be used in a sprayer, said sprayer preferably being a hand operated sprayer, for example a trigger sprayer, the sprayer more preferably comprising a fluid container being manually squeezable, a sprayer with a pressurized fluid storage container or a manually actuable pumping device, or in an electrically driven sprayer.

Pin bending and compression are problems that occur during the manufacturing process of spray nozzles having an exit end with a diameter ($d_{max}$) smaller than 0.25 mm, in particular smaller than 0.2 mm and even smaller than 0.15 mm. Therefore, high precision is required during the assembly of the pin 22 and nozzle cup 20. Thus, the spray nozzle 2 is produced by a precise injection molding process. In order to form the nozzle cup 20, the pin 22 (molding tool) is centered in a counter tool by an autopositioning process. The tapering, i.e. conical shape of the pin 22 facilitates centering of the molding tool in the counter tool as compared to a nozzle having a cylindrical pin. In addition, a conical molding tool (pin) is more robust than a cylindrical one. Further, in order to provide an edge surrounding the exit end 56 with a radius being smaller than 0.03 mm, preferably smaller than 0.02 mm micro erosion is applied for the tool manufacturing.

COMPARISON EXPERIMENTS

Example of the invention:

Outlet channel tapering (constant degree of tapering);

Diameter of exit end of the outlet channel ($d_{max}$): 0.14 mm;
Number of feeding channels: 2;
Symmetric arrangement of the feeding channels;
Angle ($\beta$): 110°;
Ratio of the sum of the cross-sectional areas of the feeding channels at their exit ends to the cross-sectional area of the exit end of the outlet channel: 1.7;
Ratio of the diameter ($d_s$) of the swirl chamber to the diameter ($d_{max}$) of the exit end: 3.4; Material: Polyoxymethylene;
Made by an injection molding process;
Fluid: water;
Flow rate: 0.24 g/s;
Propellant: nitrogen at 9 bar

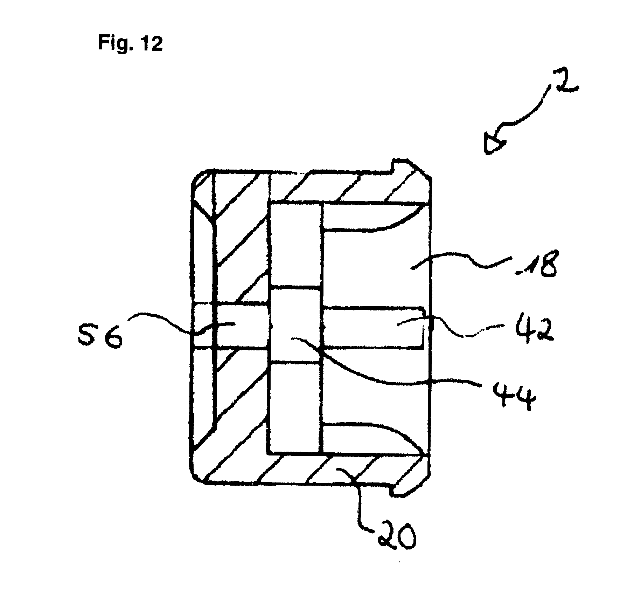

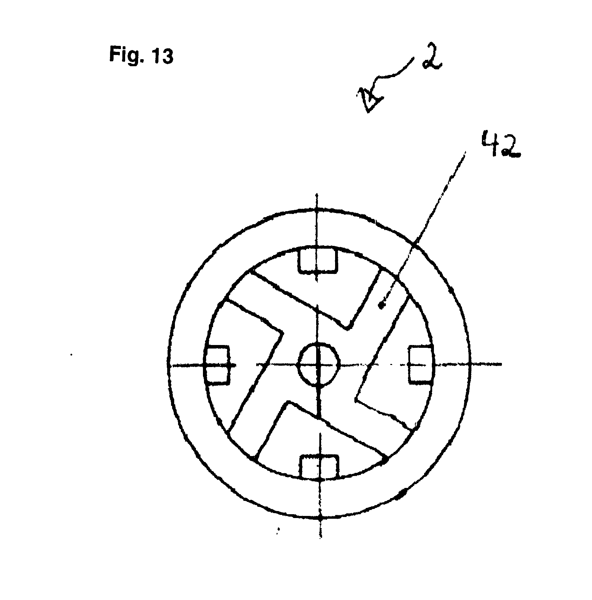

Comparative example (cf. Fig. 12 and 13):

Supplier: Coster T.E. S.p.A.;
Spray nozzle code: V06.203;
Number of feeding channels: 4
Outlet channel with cylindrical shape (not tapering);
Diameter of exit end of the outlet channel ($d_{max}$): 0.3 mm;
Length of the outlet channel: 0.2 mm;
Cross section of the feeding channels: 0.20 mm x 0.25 mm;
Fluid: water;
Flow rate: 0.30 g/s;
Propellant: nitrogen at 9 bar

A spray nozzle according to the invention (example of the invention) and a spray nozzle according to the state of the art (comparative example, cf. Fig. 12 and 13) were compared with respect to their efficiency ($\eta$). Water was applied as a fluid in connection with nitrogen at 9 bar as a compressed gas propellant.

Both nozzles were modeled and evaluated through the Yule and Dunkley efficiency equation for a pressure-swirl atomizer using the following equation:

$$\eta = 600\sigma/(\Delta p \cdot D_{32})$$

with

$\eta$=efficiency;

$\sigma$=surface tension fluid/air [N/m] ($\sigma_{water}$=0.036 N/m);

$\Delta$p=pressure drop [MPa];

$D_{32}$=Sauter Mean Diameter [$\mu$m].

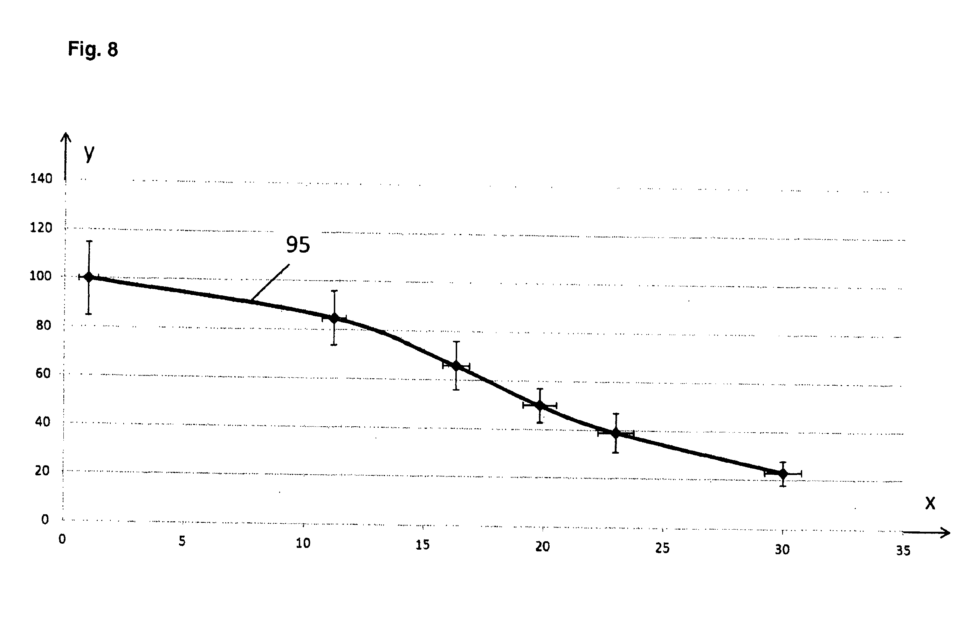

In swirl atomizers with given pressure and fluid characteristics, i.e. surface tension, the particle size ($D_{32}$) is dependent on the rotational velocity ($v_y$). In order to calculate the nozzle efficiency ($\eta$) via the Yule and Dunkley efficiency equation at known rotational velocity ($v_y$) a correlation curve of rotational velocity ($v_y$) and particle size ($D_{32}$) was determined by FE-analysis (Fig. 8). In Fig. 8, particle size in $\mu$m (y-axis) is plotted against rotational velocity in m/s (x-axis).

Graph 95 shown in Fig. 8 facilitates determining the particle size ($D_{32}$) obtained with a certain nozzle from rotational velocity ($v_y$) obtained from simulation and, thus, to calculate the respective nozzle efficiency ($\eta$). In other words, by simulating the rotational velocity ($v_y$) of a certain nozzle the respective particle size ($D_{32}$) and efficiency ($\eta$) can be determined.

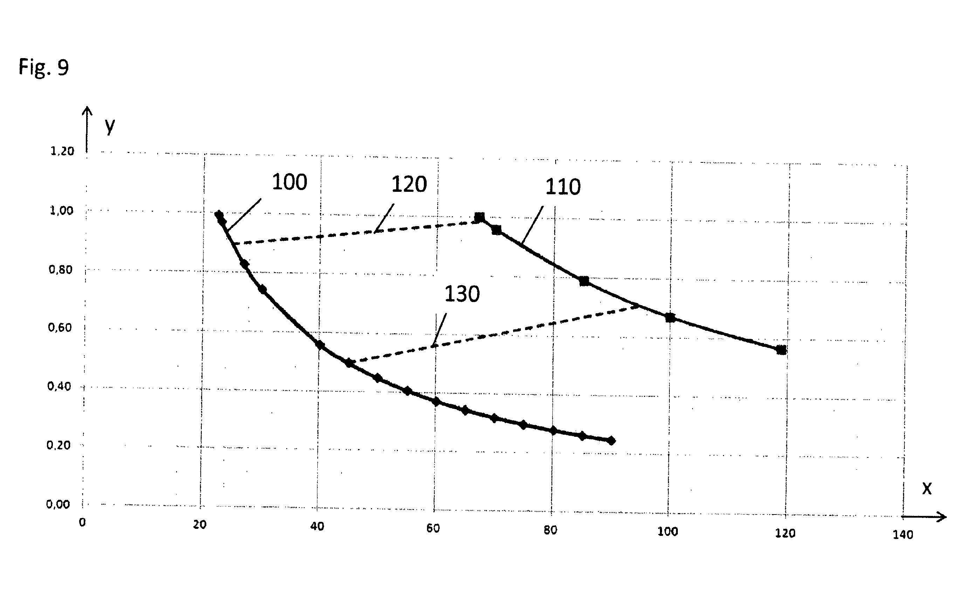

In Fig. 9 nozzle efficiency $\eta$ (y-axis) is plotted against particle size $D_{32}$ (x-axis) in accordance with the Yule and Dunkley efficiency equation. Graph 100 shows the nozzle efficiency - particle size dependency at 9 bar and graph 110 shows the respective efficiency - particle size dependency at 3 bar. Dotted line 120 displays this correlation for the spray nozzle according to the example of the invention and dotted line 130 for the spray nozzle of the comparative example. Fig. 9 clearly shows that the spray nozzle according to the example of the invention leads to improved efficiency with respect to the comparative example.

Ratio of the diameter ($d_s$) of the swir1 chamber to the diameter ($d_{max}$) of the exit end of the outlet channel:

A ratio of the diameter ($d_s$) of the swirl chamber to the diameter ($d_{max}$) of the exit end of the outlet channel of 3.4 resulted in a rotational velocity ($v_y$) of 31 m/s which corresponds to a particle size ($D_{32}$) of 23 $\mu$m ($\eta$=0.98) in accordance with the correlation shown in graph 95 (cf. Fig. 8). Ratios higher than 3.5 and lower than 2.5 resulted in a rotational velocity ($v_y$) which did not exceed 27 m/s (corresponding to a particle size ($D_{32}$) of about 58 $\mu$m). According to the Yule and Dunkley efficiency equation this corresponds to an efficiency decrease of nearly 30%, from $\eta$=0.98 to $\eta$=0.72.

Angle ($\beta$) of the outlet channel:

An angle ($\beta$) included in the inner face of the outlet channel varying between 80° and 120° has shown an increased efficiency compared to an angle ($\beta$) outside that range. Within that range a particle size ($D_{32}$) of 23 $\mu$m (for $\beta$=110°) to 40 $\mu$m has been achieved. A particle size ($D_{32}$) of 23 $\mu$m corresponds to a rotational velocity ($v_y$) of 31 m/s and efficiency ($\eta$) of 0.98. Outside that range, i.e. for an angle ($\beta$) of 70° and an angle ($\beta$) of 130°, for both angles the efficiency was about 15% lower than the best values achieved inside that range ($\beta$=110°).

Ratio of the cross-sectional areas of the feeding channels to the cross-sectional area of the exit end:

A ratio of the sum of cross-sectional areas of the feeding channels at their exit ends to the cross-sectional area of the exit end of the outlet channel chosen between 1.5 and 2.7 resulted in an increased efficiency compared to a ratio outside of that range. A ratio of 1.7 showed a rotational velocity ($v_y$) of 31 m/s with a respective particle size ($D_{32}$) of 23 $\mu$m and an efficiency ($\eta$) of 0.98. In contrast thereto, a spray nozzle having a ratio between 1 and 1.4 or between 2.8 and 3.2 showed a rotational velocity ($v_y$) of about 27 m/s with a respective particle size ($D_{32}$) of about 30 $\mu$m and an efficiency ($\eta$) of about 0,75. Thus, a decrease of efficiency of about 20% occured.

Number of feeding channels:

Provision of two feeding channels in a symmetrical arrangement showed the highest efficiency ($\eta$=0.98). A spray nozzle according to the invention having three feeding channels in a symmetrical arrangement showed a decrease of efficiency (q) of about 6%.

Constant degree of tapering:

An outlet channel with a constant degree of tapering provided an average efficiency ($\eta$) of 0.98 based on a particle size ($D_{32}$) of 23 $\mu$m. In contrast thereto, the spray nozzle according to the comparative example showed an efficiency ($\eta$) of 0.5 based on a particle size ($D_{32}$) of 46 $\mu$m.

Particle size distribution:

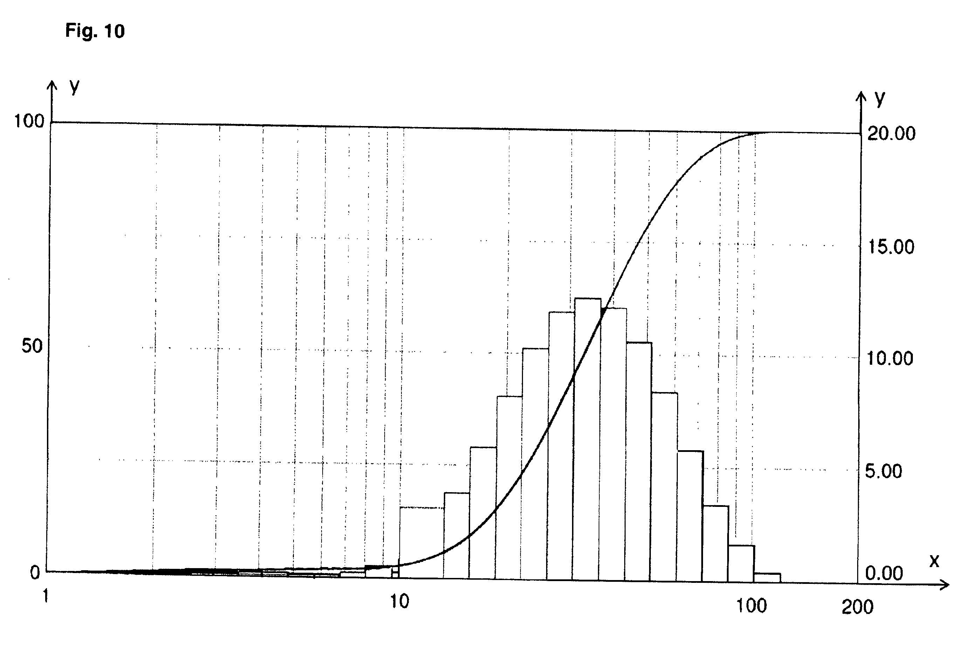

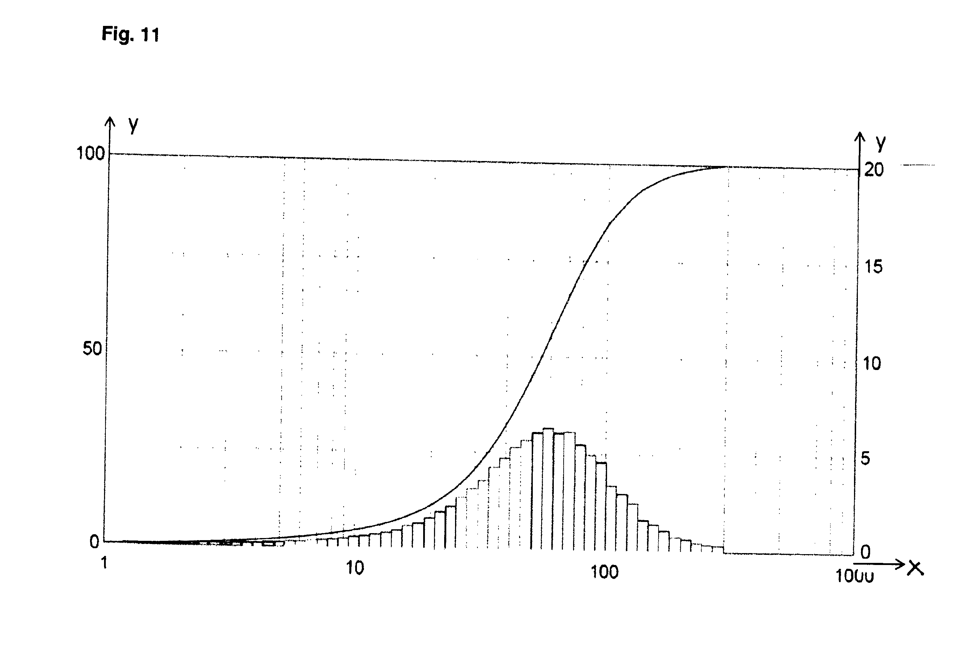

Fig. 10 shows the particle size distribution of the spray nozzle of the example of the invention, whereas Fig. 11 shows the particle size distribution of the spray nozzle of the comparative example. The cumulative volume in % (y-axis on the left side) and the volume frequency in % (y-axis on the right side) are plotted against the particle diameter in $\mu$m (x-axis), respectively. The spray nozzle according to the example of the invention (Fig. 10) resulted in a particle size ($D_{32}$) of 23 $\mu$m ($\eta$=0.98) with an average percentage of particles having a diameter smaller than 10 $\mu$m (%< 10 $\mu$m) of 1%. In contrast thereto, the spray nozzle according to the comparative example (Fig. 11) resulted in a particle size ($D_{32}$) of 30 $\mu$m ($\eta$=0.75) with an average percentage of particles having a diameter smaller than 10 $\mu$m (%< 10 $\mu$m) of about 4%. Further, the spray nozzle according to the example of the invention resulted in a narrower particle size distribution of Dv(10)-Dv(90)=46, whereas the comparative example showed a particle size distribution of Dv(10)-Dv(90)=101. Thus, the spray pattern of the nozzle according to the invention was improved as compared to the comparative example.

Spray force measurement:

The spray force of the spray nozzle according to the example of the invention measured at a distance of 23 cm on a plate having a diameter of about 14.5 cm was between 13 N and 20 N. Thus, the spray nozzle according to the example of the invention provides relatively small particles at relatively high spray force.

Anti-clogging:

The spray nozzle according to the example of the invention showed a clogging performance as good as the comparative example.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1.  Spray nozzle (2) for dispensing a fluid comprising a first element (20), preferably a nozzle cup (20), and a second element (22), preferably a pin (22), said first and second element (20, 22) forming an assembly (80) comprising a fluid chamber (40), preferably a ring chamber, for receiving the fluid, at least one feeding channel (42) for feeding the fluid from the fluid chamber (40) radially inward into a swirl chamber (44) through exit end (46) of said feeding channel (42) and an outlet channel (18) with an entrance end (54) facing the swirl chamber (44) and an exit end (56) for discharging the fluid to the environment (58) of the spray nozzle (2), the outlet channel (18) tapers in the flow direction of the fluid and the degree of tapering is either constant in the flow direction, or the degree of tapering decreases in the flow direction and the spray nozzle is **characterized in that** the ratio of the sum of the cross-sectional areas of the at least one feeding channel (42) at their exit end (46) to the cross-sectional area of the exit end (56) of the outlet channel (18) is between 1.5 and 2.7

2.  Spray nozzle (2) according to claim 1, **characterized in that**
    the exit end (56) has a maximum diameter ($d_{max}$) between about 0.1 mm and about 0.8 mm, preferably between about 0.1 mm and about 0.25 mm, more preferably between about 0.1 mm and about 0.2 mm, still more preferably between about 0.12 mm and about 0.15 mm.

3.  Spray nozzle (2) according to claim 1 or 2, **characterized in that**
    the inner face (62) of the outlet channel (18) includes an angle ($\beta$), said angle ($\beta$) varying between about 70° and about 130°, preferably between about 80° and about 120°, more preferably between about 80° and about 110°.

4.  Spray nozzle (2) according to any of the preceding claims, **characterized in that**
    the feeding channel (42) comprises a first section (48) and a second section (50) following the first section (48) in the flow direction and abutting the swirl chamber (44), the width (w1) of the first section (48) decreasing in the flow direction and the width (w2) of the second section (50) being constant or decreasing to a lesser extent in the flow direction.

5.  Spray nozzle (2) according to claim 4, **characterized in that**
    the length (1) of the second section (50) in the flow direction is equal to or smaller than the width (w2) of the second section (50) or/and the height (h) of the first or/and second section (48; 50) is decreasing in the flow direction or/and the width (w2) of the second section (50) is equal to the height (h) of the second section (50).

6.  Spray nozzle (2) according to any of the preceding claims, **characterized in that**
    the ratio of the diameter ($d_s$) of the swirl chamber (44) to the diameter ($d_{max}$) of the exit end (56) is about 2.5 to about 3.5.

7.  Spray nozzle (2) according to any of the preceding claims, **characterized in that**
    the ratio of the sum of the cross-sectional areas of the at least one feeding channel (42) at their exit end (46) to the cross-sectional area of the exit end (56) of the outlet channel (18) is between 1.7 and 2.6.

8.  Spray nozzle (2) according to any of the preceding claims, **characterized in that**
    the bottom (26) of the first element (20) exerts a pretension against the flow direction of the fluid of about 0.5 N to about 1.5 N, preferably of about 1 N.

9.  Spray nozzle (2) according to any of the preceding claims, **characterized in that**
    the bottom (26) of the first element (20) is conical in longitudinal direction (6) forming with the second element (22) a contact area which is defined by the penetration of the second element (22) during the assembly, which generate pretension between the first element (20) and the second element (22) due slightly bending the bottom (26) of the first element (20) in longitudinal direction (4).

10. Spray nozzle (2) according to any of the preceding claims, **characterized in that** one of the first and second element (20, 22) comprises an elastic portion, the elastic portion being elastically deformed by the other element (22, 20) when the elements (20, 22) are assembled, the protrusions (28) or/and the section of the first element (20) carrying the protrusions (28) preferably forming the elastic portion.

11. Spray nozzle (2) according to any of the preceding claims, **characterized in that** the first element (20) and the second element (22) are connected via a flexible connecting piece (38), preferably a strip, the connecting piece (38) more preferably being integrally formed or molded with the first and second element (20, 22).

12. Spray nozzle (2) according to one of claims 1 to 10, **characterized in that** an outlet layer (64) with a first hole (66), a channel layer (68) with a second hole (70) and slots (72) and an inlet layer (74) with holes (76) are provided, said layers (64, 68, 74) being sandwiched such that the first hole (66) forms the outlet channel (18), the second hole (70) forms the swirl chamber (44), the slots (72) form the feeding channels (42) and the holes (76) in the inlet layer (74) form inlet holes for feeding the fluid from the fluid chamber (40) into the feeding channels (42), the layers (64, 68, 74) preferably being separable from each other or/and each of the layers (64, 68, 74) preferably being replaceable.

13. Spray nozzle (2) according to claim 12, **characterized in that** there is provided an overlapping area (78) between the inlet holes and the feeding channels (42), the size of the overlapping area (78) or/and the distance between the overlapping area (78) and the swirl chamber (44) preferably being adjustable, the inlet layer (74) and the channel layer (68) more preferably being moveable, most preferably rotatable, relative to each other in order to adjust the size of the overlapping area (78) or/and the distance between the overlapping area (78) and the swirl chamber (44).

14. Spray nozzle (2) according to any of the preceding claims, **characterized in that** the spray nozzle (2) is made of a plastic material selected from the following list: polyoxymethylene, polypropylene, polyethylene, polystyrene, acrylonitrile butadiene styrene, silicone, polyamide, polyethylene terephthalate, an elastomer or mixtures thereof.

15. Sprayer comprising a spray nozzle (2) according to one of the preceding claims, said sprayer preferably being a hand operated sprayer, the sprayer more preferably comprising a fluid container being manually squeezable, a sprayer with a pressurized fluid storage container or a manually actuable pumping device, or an electrically driven sprayer.

**Patentansprüche**

1. Sprühdüse (2) zum Abgeben eines Fluids, umfassend ein erstes Element (20), vorzugsweise einen Düseneinsatz (20), und ein zweites Element (22), vorzugsweise einen Stift (22), wobei das erste und das zweite Element (20, 22) eine Anordnung (80) bilden, umfassend eine Fluidkammer (40), vorzugsweise eine Ringkammer, um das Fluid aufzunehmen, mindesten einen Zuführkanal (42) zum Zuführen des Fluids aus der Fluidkammer (40) radial nach innen in eine Wirbelkammer (44) durch das Austrittsende (46) des Zuführkanals (42) und einen Auslasskanal (18) mit einem Eintrittsende (54), das der Wirbelkammer (44) gegenüberliegt, und einem Austrittsende (56) zum Ablassen des Fluids an die Umgebung (58) der Sprühdüse (2), wobei sich der Auslasskanal (18) in die Strömungsrichtung des Fluids verjüngt und der Verjüngungsgrad in der Strömungsrichtung entweder konstant ist oder der Verjüngungsgrad in der Strömungsrichtung abnimmt und die Sprühdüse **dadurch gekennzeichnet ist, dass** das Verhältnis der Summe der Querschnittsbereiche des mindestens einen Zuführkanals (42) an seinem Austrittsende (46) zu dem Querschnittsbereich des Austrittsendes (56) des Auslasskanals (18) zwischen 1,5 und 2,7 beträgt.

2. Sprühdüse (2) nach Anspruch 1, **dadurch gekennzeichnet, dass** das Austrittsende (56) einen maximalen Durchmesser ($d_{max}$) zwischen etwa 0,1 mm und etwa 0,8 mm, vorzugsweise zwischen etwa 0,1 mm und etwa 0,25 mm, mehr bevorzugt zwischen etwa 0,1 mm und etwa 0,2 mm, noch mehr bevorzugt zwischen etwa 0,12 mm und etwa 0,15 mm aufweist.

3. Sprühdüse (2) nach Anspruch 1 oder 2, **dadurch gekennzeichnet, dass** die innere Fläche (62) des Auslasskanals (18) einen Winkel ($\beta$) aufweist, wobei der Winkel ($\beta$) zwischen etwa 70° und etwa 130°, vorzugsweise zwischen etwa 80° und etwa 120°, mehr bevorzugt zwischen etwa 80° und etwa 110° variiert.

4. Sprühdüse (2) nach einem der vorstehenden Ansprüche, **dadurch gekennzeichnet, dass** der Zuführkanal (42) einen ersten Abschnitt (48) und einen zweiten Abschnitt (50) nach dem ersten Abschnitt (48) in der Strömungsrichtung und an die Wirbelkammer (44) angrenzend umfasst, wobei die Breite (w1) des ersten Abschnitts (48) in der Strömungsrichtung abnimmt und die Breite (w2) des zweiten Abschnitts (50) konstant ist oder in der Strömungsrichtung in geringerem Maße abnimmt.

5. Sprühdüse (2) nach Anspruch 4, **dadurch gekennzeichnet, dass**
die Länge (1) des zweiten Abschnitts (50) in der Strömungsrichtung gleich oder kleiner als die Breite (w2) des zweiten Abschnitts (50) ist und/oder die Höhe (h) des ersten und/oder zweiten Abschnitts (48; 50) in der Strömungsrichtung abnimmt und/oder die Breite (w2) des zweiten Abschnitts (50) gleich der Höhe (h) des zweiten Abschnitts (50) ist.

6. Sprühdüse (2) nach einem der vorstehenden Ansprüche, **dadurch gekennzeichnet, dass**
das Verhältnis des Durchmessers ($d_s$) der Wirbelkammer (44) zu dem Durchmesser ($d_{max}$) des Austrittsendes (56) etwa 2,5 bis etwa 3,5 beträgt.

7. Sprühdüse (2) nach einem der vorstehenden Ansprüche, **dadurch gekennzeichnet, dass**
das Verhältnis der Summe der Querschnittsbereiche des mindestens einen Zuführkanals (42) an ihrem Austrittsende (46) zu dem Querschnittsbereich des Austrittsendes (56) des Auslasskanals (18) zwischen 1,7 und 2,6 beträgt.

8. Sprühdüse (2) nach einem der vorstehenden Ansprüche, **dadurch gekennzeichnet, dass**
das Unterteil (26) des ersten Elements (20) eine Vorspannung gegen die Strömungsrichtung des Fluids von etwa 0,5 N bis etwa 1,5 N, vorzugsweise etwa 1 N ausübt.

9. Sprühdüse (2) nach einem der vorstehenden Ansprüche, **dadurch gekennzeichnet, dass**
das Unterteil (26) des ersten Elements (20) in der Längsrichtung (6) kegelförmig ist und mit dem zweiten Element (22) eine Kontaktfläche bildet, die durch die Penetration des zweiten Elements (22) während des Zusammenbaus definiert ist, sodass eine Vorspannung zwischen dem ersten Element (20) und dem zweiten Element (22) aufgrund einer leichten Biegung des Unterteils (26) des ersten Elements (20) in der Längsrichtung (4) erzeugt wird.

10. Sprühdüse (2) nach einem der vorstehenden Ansprüche, **dadurch gekennzeichnet, dass**
eines des ersten und des zweiten Elements (20, 22) einen elastischen Abschnitt umfasst, der von dem anderen Element (22, 20) elastisch verformt wird, wenn die Elemente (20, 22) zusammengebaut werden, wobei die Vorsprünge (28) und/oder der Abschnitt des ersten Elements (20), der die Vorsprünge (28) trägt, den elastischen Abschnitt bilden.

11. Sprühdüse (2) nach einem der vorstehenden Ansprüche, **dadurch gekennzeichnet, dass**
das erste Element (20) und das zweite Element (22) über ein flexibles Verbindungsstück (38), vorzugsweise einen Streifen verbunden sind, wobei das Verbindungsstück (38) mehr bevorzugt mit dem ersten und dem zweiten Element (20, 22) einstückig geformt oder gegossen ist.

12. Sprühdüse (2) nach einem der Ansprüche 1 bis 10, **dadurch gekennzeichnet, dass**
eine Auslassschicht (64) mit einer ersten Öffnung (66), eine Kanalschicht (68) mit einer zweiten Öffnung (70) und Schlitzen (72) und eine Einlassschicht (74) mit Öffnungen (76) bereitgestellt sind, wobei die Schichten (64, 68, 74) derart in Sandwichform angeordnet sind, dass die erste Öffnung (66) den Auslasskanal (18) bildet, die zweite Öffnung (70) die Wirbelkammer (4) bildet, die Schlitze (72) die Zuführkanäle (42) bilden und die Öffnungen (76) in der Einlassschicht (74) Einlassöffnungen zum Zuführen des Fluids aus der Fluidkammer (40) in die Zuführkanäle (42) bilden, wobei die Schichten (64, 68, 74) vorzugsweise voneinander trennbar sind und/oder jede der Schichten (64, 68, 74) vorzugsweise austauschbar ist.

13. Sprühdüse (2) nach Anspruch 12, **dadurch gekennzeichnet, dass**
ein Überlappungsbereich (78) zwischen den Einlassöffnungen und den Zuführkanälen (42) bereitgestellt ist, die Größe des Überlappungsbereichs (78) und/oder der Abstand zwischen dem Überlappungsbereich (78) und der Wirbelkammer (44) vorzugsweise einstellbar ist, die Einlassschicht (74) und die Kanalschicht (68) mehr bevorzugt beweglich, am meisten bevorzugt drehbar zueinander sind, um die Größe des Überlappungsbereichs (78) und/oder den Abstand zwischen dem Überlappungsbereich (78) und der Wirbelkammer (44) einzustellen.

14. Sprühdüse (2) nach einem der vorstehenden Ansprüche, **dadurch gekennzeichnet, dass**
die Sprühdüse (2) aus einem Kunststoffmaterial hergestellt ist, das aus der folgenden Liste ausgewählt ist: Polyoxymethylen, Polypropylen, Polyethylen, Polystyrol, Acrylnitrilbutadienstyrol, Silikon, Polyamid, Polyethylenterephthalat, einem Elastomer oder Mischungen davon.

15. Sprüher, umfassend eine Sprühdüse (2) nach einem der vorstehenden Ansprüche, wobei der Sprüher vorzugsweise ein handbetriebener Sprüher ist, wobei der Sprüher mehr bevorzugt einen Fluidbehälter, der manuell zusammendrückbar ist, einen Sprüher mit einem druckbeaufschlagten Fluidspeicherbehälter oder eine manuell betätigbare Pumpenvorrichtung oder einen elektrisch angetriebenen Sprüher umfasst.

**Revendications**

1. Buse de pulvérisation (2) pour distribuer un fluide, comprenant un premier élément (20), de préférence une coupelle de buse (20), et un deuxième élément (22), de préférence une goupille (22), lesdits premier et deuxième éléments (20, 22) formant un ensemble (80) comprenant une chambre à fluide (40), de préférence une chambre annulaire, pour recevoir le fluide, au moins un canal d'alimentation (42) pour alimenter le fluide de la chambre à fluide (40) en sens radial vers l'intérieur dans une chambre de tourbillonnement (44) à travers une extrémité de sortie (46) dudit canal d'alimentation (42) et un canal de sortie (18) avec une extrémité d'entrée (54) faisant face à la chambre de tourbillonnement (44) et une extrémité de sortie (56) permettant d'évacuer le fluide vers l'environnement (58) de la buse de pulvérisation (2), le canal de sortie (18) s'effile dans la direction d'écoulement du fluide et le degré d'effilement est ou constant dans la direction d'écoulement, ou le degré d'effilement diminue dans la direction d'écoulement et la buse de pulvérisation est **caractérisée en ce que** le rapport de la somme des aires en coupe transversale de l'au moins un canal d'alimentation (42) au niveau de leur extrémité de sortie (46) sur l'aire en coupe transversale de l'extrémité de sortie (56) du canal de sortie (18) est compris entre 1,5 et 2,7

2. Buse de pulvérisation (2) selon la revendication 1, **caractérisée en ce que** l'extrémité de sortie (56) a un diamètre maximum ($d_{max}$) compris entre environ 0,1 mm et environ 0,8 mm, de préférence entre environ 0,1 mm et environ 0,25 mm, plus préférablement entre environ 0,1 mm et environ 0,2 mm, encore plus préférablement entre environ 0,12 mm et environ 0,15 mm.

3. Buse de pulvérisation (2) selon la revendication 1 ou 2, **caractérisée en ce que** la face interne (62) du canal de sortie (18) inclut un angle (β), ledit angle (β) variant entre environ 70° et environ 130°, de préférence entre environ 80° et environ 120°, plus préférablement entre environ 80° et environ 110°.

4. Buse de pulvérisation (2) selon l'une quelconque des revendications précédentes, **caractérisée en ce que** le canal d'alimentation (42) comprend une première section (48) et une deuxième section (50) suivant la première section (48) dans la direction d'écoulement et venant en butée contre la chambre de tourbillonnement (44), la largeur (w1) de la première section (48) diminuant dans la direction d'écoulement et la largeur (w2) de la deuxième section (50) étant constante ou diminuant dans une moindre mesure dans la direction d'écoulement.

5. Buse de pulvérisation (2) selon la revendication 4, **caractérisée en ce que** la longueur (1) de la deuxième section (50) dans la direction d'écoulement est égale ou inférieure à la largeur (w2) de la deuxième section (50) ou/et la hauteur (h) de la première ou/et la deuxième section (48 ; 50) diminue dans la direction d'écoulement ou/et la largeur (w2) de la deuxième section (50) est égale à la hauteur (h) de la deuxième section (50).

6. Buse de pulvérisation (2) selon l'une quelconque des revendications précédentes, **caractérisée en ce que** le rapport du diamètre ($d_s$) de la chambre de tourbillonnement (44) sur le diamètre ($d_{max}$) de l'extrémité de sortie (56) va d'environ 2,5 à environ 3,5.

7. Buse de pulvérisation (2) selon l'une quelconque des revendications précédentes, **caractérisée en ce que** le rapport de la somme des aires en coupe transversale de l'au moins un canal d'alimentation (42) à leur extrémité de sortie (46) sur l'aire en coupe transversale de l'extrémité de sortie (56) du canal de sortie (18) est compris entre 1,7 et 2,6.

8. Buse de pulvérisation (2) selon l'une quelconque des revendications précédentes, **caractérisée en ce que** le fond (26) du premier élément (20) exerce une mise en tension préalable à l'encontre de la direction d'écoulement du fluide d'environ 0,5 N à environ 1,5 N, de préférence d'environ 1 N.

9. Buse de pulvérisation (2) selon l'une quelconque des revendications précédentes, **caractérisée en ce que** le fond (26) du premier élément (20) est conique dans la direction longitudinale (6) formant avec le deuxième élément (22) une zone de contact qui est définie par la pénétration du deuxième élément (22) durant l'assemblage, ce qui génère une mise en tension préalable entre le premier élément (20) et le deuxième élément (22) due à un léger cintrage du fond (26) du premier élément (20) dans la direction longitudinale (4).

10. Buse de pulvérisation (2) selon l'une quelconque des revendications précédentes, **caractérisée en ce que** un des premier et deuxième éléments (20, 22) comprend une partie élastique, la partie élastique étant élastiquement déformée par l'autre élément (22, 20) lorsque les éléments (20, 22) sont assemblés, les parties saillantes (28) ou/et la section du premier élément (20) portant les parties saillantes (28) formant de préférence la partie élastique.

**11.** Buse de pulvérisation (2) selon l'une quelconque des revendications précédentes, **caractérisée en ce que**
le premier élément (20) et le deuxième élément (22) sont reliés par l'intermédiaire d'une pièce de liaison souple (38), de préférence une bande, la pièce de liaison (38) étant plus préférablement formée en une seule pièce ou moulée avec les premier et deuxième éléments (20, 22).

**12.** Buse de pulvérisation (2) selon l'une des revendications 1 à 10, **caractérisée en ce que**
une couche de sortie (64) avec un premier trou (66), une couche de canal (68) avec un deuxième trou (70) et des encoches (72) et une couche d'entrée (74) avec des trous (76) sont fournies, lesdites couches (64, 68, 74) étant intercalées de telle sorte que le premier trou (66) forme le canal de sortie (18), le deuxième trou (70) forme la chambre de tourbillonnement (44), les encoches (72) forment les canaux d'alimentation (42) et les trous (76) dans la couche d'entrée (74) forment des trous d'entrée pour alimenter le fluide de la chambre à fluide (40) dans les canaux d'alimentation (42), les couches (64, 68, 74) étant de préférence séparables l'une de l'autre ou/et chacune des couches (64, 68, 74) étant de préférence remplaçable.

**13.** Buse de pulvérisation (2) selon la revendication 12, **caractérisée en ce que**
on fournit une zone de chevauchement (78) entre les trous d'entrée et les canaux d'alimentation (42), la taille de la zone de chevauchement (78) ou/et la distance entre la zone de chevauchement (78) et la chambre de tourbillonnement (44) étant de préférence réglable, la couche d'entrée (74) et la couche de canal (68) étant plus préférablement mobiles, le plus préférablement rotatives, l'une par rapport à l'autre afin d'ajuster la taille de la zone de chevauchement (78) ou/et la distance entre la zone de chevauchement (78) et la chambre de tourbillonnement (44).

**14.** Buse de pulvérisation (2) selon l'une quelconque des revendications précédentes, **caractérisée en ce que**
la buse de pulvérisation (2) est constituée d'un matériau en plastique choisi parmi la liste suivante : polyoxyméthylène, polypropylène, polyéthylène, polystyrène, acrylonitrile butadiène styrène, silicone, polyamide, polyéthylène téréphtalate, un élastomère ou leurs mélanges.

**15.** Pulvérisateur comprenant une buse de pulvérisation (2) selon l'une des revendications précédentes, ledit pulvérisateur étant de préférence un pulvérisateur actionné manuellement, le pulvérisateur comprenant plus préférablement un récipient à fluide compressible manuellement, un pulvérisateur avec un récipient de stockage de fluide sous pression ou un dispositif de pompe actionnable manuellement, ou un pulvérisateur à entraînement électrique.

**Fig. 1**

**Fig. 2**

**Fig. 3**

**Fig. 4**

**Fig. 5**

## Fig. 6

Fig. 7

Fig. 8

Fig. 9

Fig. 10

**Fig. 11**

EP 2 570 193 B1

Fig. 12

56

18

42

44

2

20

Fig. 13